# EUROPEAN PATENT APPLICATION

(11) **EP 4 364 837 A1**
(43) Date of publication of application: **08.05.2024**
(21) Application number: 22205396.9
(22) Date of filing: 03.11.2022
(51) Int. Cl.: B01J 13/00

(54) **METHOD FOR PREPARING A PERSONAL CARE COMPOSITION, APPARATUS, AND PERSONAL CARE COMPOSITION OBTAINED THEREOF**

(71) Applicant: Wella Germany GmbH, 64295 Darmstadt (DE)
(72) Inventor: HEIL, Frank, 64295 Darmstadt (DE); KOVACS, Tibor Z., 69151 Neckargemünd (DE); RUPPERT, Stephanie, 64295 Darmstadt (DE); HUBER, Markus, 64295 Darmstadt (DE); SCHOLZ, Didier, 64295 Darmstadt (DE); HOLSTEIN, Ralf, 64295 Darmstadt (DE); KOETT, Tobias, 64295 Darmstadt (DE)
(74) Representative: Bandpay & Greuter

(57) **Abstract**

A method for preparing a personal care composition, a personal care composition obtained thereof, and an apparatus suitable for implementing the method are provided.

## Description

### Field

The presently claimed invention relates to a method for preparing a personal care composition, a personal care composition obtained thereof, and an apparatus suitable for implementing the method.

### Background

A wide variety of cosmetic compositions e.g., personal care compositions are available to the consumers. Many of them are emulsions and are prepared by mixing different compounds, in order to provide compositions having satisfactory properties and benefits to the consumer. For example, without wishing to be exhaustive, suitable compounds may be chosen from the group consisting of polyols, fatty substances, surfactants, direct dyes, lipids, conditioning agents, anti-dandruff agents, thickening and rheology-modifying agents, suspending agents, solubilizing agents, preservatives, antioxidants, UV-filters and/or sunscreens, perfume or fragrance, moisturizing and humecting agents.

Formulation is a complex science, as it requires mixing the compounds at the right time, in the right order, under the right conditions, in order to obtain compositions having the expected structure, properties and benefits. The formulator should carefully choose the apparatus and the method, knowing that the compounds should be sequentially added under different conditions.

Various methods are known. See for example the US patent 10,695,274 B2.

Even though various methods are known; they are not always suitable for preparing the different types of personal care compositions. Particularly, known methods are not entirely satisfactory, when it comes to the preparation of personal care compositions comprising a gel network system.

A gel network system is obtained, upon mixing at least one fatty alcohol and at least one non-ionic surfactant. For example, the at least one fatty alcohol may be selected from the group consisting of linear or branched C₁₄ to C₃₀ fatty alcohols, and the at least one non-ionic surfactant may be selected from the group consisting of polyoxyethylene C₈ to C₃₀ alkyl ethers. For example, the at least one fatty alcohol may be present in an amount in the range of ≥ 2.5 to ≤ 20.0 wt.-%, and the at least one non-ionic surfactant may be present in an amount in the range of ≥ 0.2 to ≤ 5.0 wt.-%, more preferably in the range of ≥ 0.5 to ≤ 4 wt.-%, even more preferably in the range of ≥ 1.0 to ≤ 2.5 wt.-%, related to the total weight of the composition.

There is a need for an improved method for preparing a personal care composition, particularly a personal care composition comprising a gel network system.

### Summary

The inventors have shown that personal care compositions, particularly personal care compositions comprising a gel network are satisfactorily prepared with the method of the presently claimed invention. Indeed, the inventors have shown that the method allows preparing a personal care composition having a stable gel network, and into which any suitable cosmetically acceptable compounds can be added, including thermosensitive compounds. This is achieved by providing a method - in which various parameters are carefully controlled, including the temperature, the shear, the flow rate, the pressure, etc. - using a suitable, specific apparatus.

Hence, in a first aspect, the presently claimed invention relates to a method for preparing a personal care composition, comprising at least the steps of:
(A) providing a recirculated aqueous stream comprising at least one fatty alcohol and at least one non-ionic surfactant; wherein the fatty alcohol and the non-ionic surfactant are comprised, in the recirculated aqueous stream, in part or all, in a gel network system;
(B) exchanging heat between the recirculated aqueous stream and a cooling media, for obtaining a cooled, recirculated aqueous stream; wherein the stream is cooled to a temperature of ≤ 45°C, preferably in the range of ≥ 15 to ≤ 45°C, more preferably in the range of ≥ 25 to ≤ 45°C;
(C) adding at least one thermosensitive compound into the cooled, recirculated aqueous stream; and
(D) obtaining a personal care composition.

In a preferred embodiment, the recirculated aqueous stream is recirculated through the reactor.

In a preferred embodiment, the at least one fatty alcohol is selected from the group consisting of linear or branched C₁₄ to C₃₀ fatty alcohols; more preferably from the group consisting of linear C₁₄ to C₃₀ fatty alcohols; even more preferably from the group consisting of cetyl alcohol, stearyl alcohol, cetearyl alcohol and behenyl alcohol.

In a preferred embodiment, the at least one non-ionic surfactant is selected from the group consisting of polyoxyethylene C₈ to C₃₀ alkyl ethers; preferably from the group consisting of polyoxyethylene C₈ to C₃₀ alkyl ethers having at least 2 ethylene oxide units, or at least 5 ethylene oxide units; more preferably from the group consisting of polyoxyethylene C₈ to C₃₀ alkyl ethers having from 10 to 50 ethylene oxide units; even more preferably from the group consisting of polyoxyethylene C₈ to C₃₀ alkyl ethers having from 15 to 30 ethylene oxide units; still preferably from the group consisting of ceteareth-25 and steareth-20.

In a preferred embodiment, the recirculated aqueous stream and the personal care composition obtained thereof comprise the at least one fatty alcohol and the at least one non-ionic surfactant in a weight ratio in the range of ≥ 10.0:0.5 to ≤ 10.0:4.0, more preferably in the range of ≥ 10.0:1.0 to ≤ 10.0:3.5; even more preferably in the range of ≥ 10.0:1.5 to ≤ 10.0:3.0.

In a preferred embodiment, the personal care composition obtained thereof comprises the at least one fatty alcohol in an amount in the range of ≥ 2.5 to ≤ 20.0 wt.-%, preferably in the range of ≥ 3 to ≤ 15 wt.-%, more preferably in the range of ≥ 4 to ≤ 10 wt.-%, related to the total weight of the composition.

In a preferred embodiment, the personal care composition obtained thereof comprises the at least one non-ionic surfactant in an amount in the range of ≥ 0.2 to ≤ 5.0 wt.-%, more preferably in the range of ≥ 0.5 to ≤ 4 wt.-%, even more preferably in the range of ≥ 1.0 to ≤ 2.5 wt.-%, related to the total weight of the composition.

In a preferred embodiment, the recirculated aqueous stream comprising at least one fatty alcohol and at least one non-ionic surfactant is provided at a temperature in the range of ≥ 50 to ≤ 65 °C.

In a preferred embodiment, the recirculated aqueous stream comprising at least one fatty alcohol and at least one non-ionic surfactant is provided at a temperature in the range of ≥ 50 to ≤ 65 °C. This range of temperature is advantageous, as it allows stabilizing the emulsion obtained.

In a preferred embodiment, the method comprises, before step (A), the step of adding the at least one fatty alcohol and the at least one non-ionic surfactant into the aqueous fluid.

In a preferred embodiment, the aqueous fluid has at a temperature in the range of ≥ 70 to ≤ 85 °C.

In a preferred embodiment, the method comprises, after adding the at least one fatty alcohol and the at least one non-ionic surfactant into the aqueous fluid, and before providing the recirculated aqueous stream of step (A), the step of pre-cooling the recirculated aqueous stream, preferably at a temperature in the range of ≥ 50 to ≤ 65 °C.

In a preferred embodiment, the method comprises the step of providing a concentrated aqueous feed stream and directly introducing it into the recirculated aqueous stream, preferably wherein the concentrated aqueous feed stream comprises sodium laureth sulfate.

In a second aspect, the presently claimed invention relates to a personal care composition, which is obtained with the method as described above and below.

In a preferred embodiment, the personal care composition is selected from the group consisting of shampoo compositions and conditioning compositions.

In a third aspect, the presently claimed invention relates to an apparatus, suitable for preparing a personal care composition as described above and below, wherein the apparatus comprises:
- a reactor for holding an aqueous liquid; and
- a recircularization loop for conveying the aqueous fluid as a recirculated aqueous stream from the reactor back to the reactor; wherein the recircularization loop comprises a pump and a heat exchanger.

### Brief description of the figure

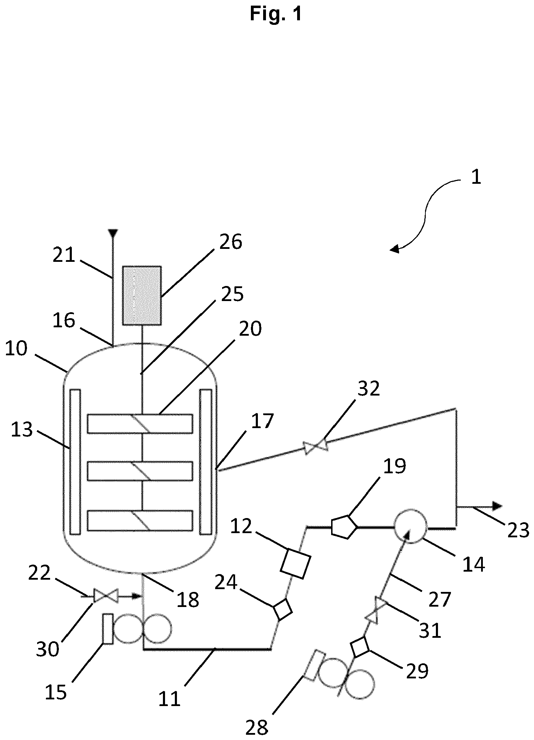

Figure 1 represents a schematic diagram of the apparatus as described above and below.

### Detailed description

Before the different aspects of the presently claimed invention, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are interchangeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

Furthermore, the ranges defined throughout the specification include the end values as well, *i.e.,* a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment, but may refer to the same embodiment. Further, as used in the following, the terms *"preferably", "more preferably*", *"even more preferably*", *"most preferably*" and *"in particular"* or similar terms are used in conjunction with optional features, without restricting alternative possibilities. Thus, features introduced by these terms are optional features and are not intended to restrict the scope of the claims in any way.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

Further, it shall be noted that the terms *"at least one", "one or more"* or similar expressions indicating that a feature or element may be present once or more than once typically will be used only once when introducing the respective feature or element. In the following, in most cases, when referring to the respective feature or element, the expressions *"at least one"* or *"one or more"* will not be repeated, non-withstanding the fact that the respective feature or element may be present once or more than once.

As used herein the term *"hair"* may be *"living", i.e.,* on a living body, or may be *"non-living", i.e.,* in a wig, hairpiece or other aggregation of non-living keratinous fibres. Mammalian, preferably human hair is preferred. However, wool, fur and other keratin fibres (keratin-containing fibres) are suitable substrates for the composition of the presently claimed invention.

By *"substantially free of"* is meant a composition comprising a compound in an amount of ≤ 0.1 wt.-% or less, preferably in an amount of ≤ 0.01 wt.-% or less, more preferably in an amount of 0 wt.-% (free of), related to the total weight of the composition.

By *"personal care composition"* is meant a composition, which is suitable for application to skin and hair, particularly to human skin and hair. For example, a personal care composition may be a shampoo and/or conditioning composition.

All percentages are by weight of the fluid to which it is referred, unless otherwise specified. Also ratios are weight ratios unless specifically stated otherwise.

### Method for preparing a personal care composition

In a first aspect, the presently claimed invention relates to a method for preparing a personal care composition, comprising at least the steps of:
(A) providing a recirculated aqueous stream comprising at least one fatty alcohol and at least one non-ionic surfactant; wherein the fatty alcohol and the non-ionic surfactant are comprised, in the recirculated aqueous stream, in part or all, in a gel network system;
(B) exchanging heat between the recirculated aqueous stream and a cooling media, for obtaining a cooled, recirculated aqueous stream; wherein the stream is cooled to a temperature of ≤ 45°C, preferably in the range of ≥ 15 to ≤ 45°C, more preferably in the range of ≥ 25 to ≤ 45°C;
(C) adding at least one thermosensitive compound into the cooled, recirculated aqueous stream; and
(D) obtaining a personal care composition.

The method is implemented with a suitable apparatus 1, as described above and below.

The method comprises the step of providing a recirculated aqueous stream comprising at least one fatty alcohol and at least one non-ionic surfactant (step A - step of providing a gelled recirculated aqueous stream).

The at least one fatty alcohol and the at least one non-ionic surfactant are suitable compounds forming a gel network system. The at least one fatty alcohol and the at least one non-ionic surfactant are therefore comprised, in the recirculated aqueous stream and the personal care composition obtained thereof, in part or all, in a gel network system.

In a preferred embodiment, the at least one fatty alcohol is selected from the group consisting of linear or branched C₁₄ to C₃₀ fatty alcohols; more preferably from the group consisting of linear C₁₄ to C₃₀ fatty alcohols; even more preferably from the group consisting of cetyl alcohol, stearyl alcohol, cetearyl alcohol and behenyl alcohol.

In a preferred embodiment, the at least one non-ionic surfactant (non-fatty non-ionic surfactant) is selected from the group consisting of polyoxyethylene C₈ to C₃₀ alkyl ethers; preferably from the group consisting of polyoxyethylene C₈ to C₃₀ alkyl ethers having at least 2 ethylene oxide units, or at least 5 ethylene oxide units; more preferably from the group consisting of polyoxyethylene C₈ to C₃₀ alkyl ethers having from 10 to 50 ethylene oxide units; even more preferably from the group consisting of polyoxyethylene C₈ to C₃₀ alkyl ethers having from 15 to 30 ethylene oxide units; still preferably from the group consisting of ceteareth-25 and steareth-20. The non-ionic surfactant is different from the at least one fatty alcohol described above and below.

In a preferred embodiment, the recirculated aqueous stream, and the personal care composition thereof, comprise the at least one fatty alcohol and the at least one non-ionic surfactant in a weight ratio in the range of ≥ 10.0:0.5 to ≤ 10.0:4.0, more preferably in the range of ≥ 10.0:1.0 to ≤ 10.0:3.5; even more preferably in the range of ≥ 10.0:1.5 to ≤ 10.0:3.0.

The at least one fatty alcohol is present in the recirculated aqueous stream, such that the personal care composition obtained thereof comprises the at least one fatty alcohol in an amount in the range of ≥ 2.5 to ≤ 20.0 wt.-%, preferably in the range of ≥ 3 to ≤ 15 wt.-%, more preferably in the range of ≥ 4 to ≤ 10 wt.-%, related to the total weight of the composition.

The at least one non-ionic surfactant is present in the recirculated aqueous stream, such that the personal care composition obtained thereof comprises at least one non-ionic surfactant in an amount in the range of ≥ 0.2 to ≤ 5.0 wt.-%, more preferably in the range of ≥ 0.5 to ≤ 4 wt.-%, even more preferably in the range of ≥ 1.0 to ≤ 2.5 wt.-%, related to the total weight of the composition.

The recirculated aqueous stream is recirculated through the reactor 10. It is advantageous to provide a method, in which the aqueous fluid is circulated through a recircularization loop 11 from the reactor 10 back to reactor 10, as it allows adding the compounds to the aqueous fluid at the right time, in the right order, under the right conditions, while keeping storing the aqueous fluid in the reactor 10. This simplifies the method, as there is no need for additional devices, for example additional reactors, for storing the premixes obtained in between.

The method also comprises the step of exchanging heat between the recirculated aqueous stream and a cooling media, for obtaining a cooled, recirculated aqueous stream (step B - cooling step).

The recirculated aqueous stream is cooled to a temperature of ≤ 45°C, preferably in the range of ≥ 15 to ≤ 45°C, more preferably in the range of ≥ 25 to ≤ 45°C. It is advantageous to cool the recirculated aqueous stream at this range of temperature, as it avoids the decomposition of any of the thermosensitive compounds added afterwards.

The recirculated aqueous stream and the cooling media are not fluidly connected. Any suitable cooling media may be used. It is advantageous to use an apparatus comprising a recircularization loop 11 equipped with a heat exchanger 12, as it allows varying and monitoring the temperature of the aqueous fluid, directly within the recircularization loop 11. It avoids any unwanted cooling during the recircularization and therefore stabilizes the temperature of the aqueous fluid, both in the reactor 10 and in the recircularization loop 11.

The method also comprises the step of adding at least one thermosensitive compound into the cooled, recirculated aqueous stream (step C - thermosensitive addition step). By *"thermosensitive compound"* is meant any compound, which may degrade or be altered when heated. Any suitable thermosensitive compounds known to the skilled person may be added. Without wishing to be exhaustive, thermosensitive compounds may be perfumes or fragrances, plant extracts, etc.

The method also comprises the step of obtaining a personal care composition (step D - final step).

Prior addition of the at least one fatty alcohol and the at least non-ionic surfactant, the method may comprise the step of providing an aqueous fluid and conveying it as a recirculated aqueous stream (step of providing an aqueous fluid).

In a preferred embodiment, the aqueous fluid essentially consists of water (i.e., about 100 wt.-%). In another preferred embodiment, the aqueous fluid comprises water and at least one additional compound. The aqueous fluid may comprise any suitable compound for incorporation into a personal care composition.

The aqueous fluid, which is hold in the reactor 10 and which circulates as the recirculated aqueous stream, may be provided at a temperature in the range of ≥ 70 to ≤ 85 °C. Any suitable heating devices may be used. In one embodiment, the aqueous fluid may be heated using a heater 13, which is located in the vicinity of the reactor 10. In another embodiment, the recirculated aqueous stream may be heated using the heat exchanger 12, which is located at any suitable position of the recircularization loop 11. It is advantageous to heat the aqueous fluid at a temperature in the range of ≥ 70 to ≤ 85 °C, as it allows adding the at least one fatty alcohol and the at least one non-ionic surfactant at a temperature being superior to the temperature of crystallization of the at least one fatty compound and the at one non-ionic surfactant.

After providing an aqueous fluid and conveying it as a recirculated aqueous stream, the method may comprise the step of adding the at least one fatty alcohol and the at least one non-ionic surfactant into the aqueous fluid (step of adding fatty alcohols and non-ionic surfactants). This step allows obtaining an ungelled aqueous fluid, and ungelled recirculated aqueous stream thereof.

After the step of adding fatty alcohols and non-ionic surfactants, and prior providing the gelled recirculated aqueous stream, the method may comprise the step of pre-cooling the ungelled aqueous fluid (precooling step). Any suitable heating devices may be used, including the heater 13, which is located in the vicinity of the reactor 10, and/or the heat exchanger 12, which is located at any suitable position of the recircularization loop 11. It is advantageous to cool the aqueous fluid at a temperature in the range of ≥ 50 to ≤ 65 °C, which is a temperature range allowing the stabilisation of the emulsion and the formation of the gel network system, upon crystallization of the at least one fatty alcohol and the at least one non-ionic surfactant.

The method may comprise the step of providing a concentrated aqueous feed stream and directly introducing it into the recirculated aqueous stream (step of providing a concentrated aqueous feed stream). In a preferred embodiment, the concentrated aqueous feed stream comprises sodium laureth sulfate.

The step of providing a concentrated aqueous feed stream may be carried at any time during the method. In a preferred embodiment, the step of providing a concentrated aqueous feed stream is carried out prior the step of adding the at least one fatty alcohol and the at least one non-ionic surfactant into the aqueous fluid.

Amongst the sulfate anionic surfactants conventionally used for preparing a personal care composition, sodium laureth sulfate is a particularly suitable surfactant. Indeed, sodium laureth sulfate exhibits superior performance, particularly in terms of foaming, cleaning and emulsifying properties. In addition, sodium laureth sulfate is rather inexpensive *versus* alternative anionic surfactants. The present step of introducing sodium laureth sulfate is advantageous, in that it improves the mixing of the concentrated aqueous feed stream with the recirculated aqueous stream, and avoids the generation of localized areas of very high viscosity

Sodium laureth sulfate is also known as sodium lauryl ether sulfate (SLES). Both expressions are interchangeably used. Sodium laureth sulfate has the following chemical formula (I):

CH₃(CH₂)₁₁(OCH₂CH₂)*ₙ*OSO₃Na (I)

wherein the indicia "n" corresponds to the number of ethoxyl groups and is in the range of ≥ 2 to ≤ 6, preferably n is 2. Sodium laureth sulfate may be prepared by ethoxylation of dodecyl alcohol, which may be derived from palm kernel oil or coconut oil. The resulting ethoxylate may therefore be converted to a half ester of sulfuric acid, which may thus be neutralized by conversion to the sodium salt.

Sodium laureth sulfate is a compound, which exhibits a specific viscosity behaviour, and which is sometimes referred to as a *"wormlike micelle-forming surfactant"* or a *"non-monotonic viscoelastic surfactant".* When the concentration of sodium laureth sulfate is of ≤ 28 wt.-% or less or ≥ 60 wt.-% or more, the material comprising it has a viscosity, which is low enough for being processed. In contrast, when the concentration of sodium laureth sulfate is in the range of > 28 to < 60 wt.-%, the viscosity of the material comprising it dramatically increases up to levels, at which the material cannot be processed anymore. A method for diluting sodium laureth sulfate from a concentrated raw material is known (see the technical brochure from the company Silverson available online at: https://www.silverson.com/us/resource-library/application-reports/dilution-of-high-active-surfactants).

In a preferred embodiment, sodium laureth sulfate is selected from the group consisting of sodium laureth-2 sulfate, sodium laureth-3 sulfate, and sodium laureth-5 sulfate. In a more preferred embodiment, sodium laureth sulfate is sodium laureth-2 sulfate.

In a preferred embodiment, the concentrated aqueous feed stream comprises sodium laureth sulfate at a concentration in the range of ≥ 60 to ≤ 80 wt.%, preferably in the range of ≥ 65 to ≤ 75 wt.%, more preferably in the range of ≥ 68 to ≤ 72 wt.%, related to the total weight of the concentrated aqueous feed stream.

In a preferred embodiment, the concentrated aqueous feed stream has a flow rate in the range of ≥ 20 to ≤ 100 kg/min, preferably in the range of ≥ 30 to ≤ 70 kg/min, more preferably in the range of ≥ 35 to ≤ 65 kg/min. This flow rate is advantageous in that it allows introducing the concentrated aqueous feed stream into the recirculated aqueous stream at a rate, which eases the solubilisation of sodium laureth sulfate.

In a preferred embodiment, the concentrated aqueous feed stream has a temperature in the range of ambient temperature, preferably in the range of ≥ 15 to ≤ 30 °C, more preferably in the range of ≥ 18 to ≤ 25 °C.

In a preferred embodiment, the concentrated aqueous feed stream is introduced into the recirculated aqueous stream for a period in the range of ≥ 1min to ≤ 1h, preferably in the range of ≥ 2 to ≤ 45 min, more preferably in the range of ≥ 3 to ≤ 30 min.

In a preferred embodiment, the pressure of the concentrated aqueous feed stream is superior to the pressure of the recirculated aqueous stream, preferably wherein the pressure of the concentrated aqueous feed stream is at least 0.4×10⁵ Pa higher than the pressure of the recirculated aqueous stream. This difference of pressure can advantageously be obtained, by providing an apparatus comprising two separated pumps, as described above and below.

In a preferred embodiment, sodium laureth sulfate is directly introduced into the recirculated aqueous stream using a homogenizer 14, preferably a rotor-stator homogenizer. The use of a homogenizer 14, preferably a rotor-stator homogenizer, is advantageous in that there is sufficient friction for ensuring that the sodium laureth sulfate is homogeneously diluted into the recirculated aqueous stream. The inventors have shown that directly introducing the concentrated aqueous feed stream into the homogenizer 14, instead of another section of the recircularization loop, improves the mixing of the concentrated aqueous feed stream with the recirculated aqueous stream, and avoids the generation of localized areas of very high viscosity.

In a preferred embodiment, once the concentrated aqueous feed stream has been added into the recirculated aqueous stream, the ratio of the weight concentrations of sodium laureth sulfate between the concentrated aqueous stream and the diluted aqueous stream is in the range of ≥ 2.0:1.0 to ≤ 5.0:1.0, preferably ≥ 3.0:1.0 to ≤ 5.0:1.0, more preferably ≥ 4.0:1.0 to ≤ 5.0:1.0.

In a preferred embodiment, the personal care composition is selected from the group consisting of shampoo compositions and conditioning compositions.

### Apparatus for preparing a diluted aqueous stream comprising sodium laureth sulfate

In a second aspect, the presently claimed invention relates to an apparatus 1, suitable for preparing a personal care composition as described above and below, wherein the apparatus 1 comprises:
- a reactor 10 for holding an aqueous liquid; and
- a recircularization loop 11 for conveying the aqueous fluid as a recirculated aqueous stream from the reactor 10 back to the reactor 10; wherein the recircularization loop 11 comprises a pump 15 and a heat exchanger 12.

The terms *"upstream"* and *"downstream"* are provided in reference to the fluid circularization through the recircularization loop.

The apparatus 1 comprises a reactor 10.

The reactor 10 may comprise at least one circumferential outer wall, delimitating an inner volume. The reactor 10 may comprise one outer wall or two outer walls (so-called double-wall reactor/tank). The inner volume may be filled with the fluids, which are supplied to/obtained in it, when carrying out the methods described herein.

The reactor 10 may be substantially cylindrical in shape and have a substantially circular cross-section. The reactor 10 may be substantially oriented in a vertical position, such that the reactor 10 comprises a substantially vertical longitudinal axis and a substantially horizontal circular cross-section. The terms *"top"* and *"bottom"* are provided in reference to a substantially cylindrical reactor 10, which is in a vertical position.

The reactor 10 may have a volume in the range of ≥ 50 to ≤ 5,000 L.

The reactor 10 may have any suitable alternative shape and dimensions.

The reactor 10 may be an open reactor or a closed reactor, preferably the reactor is a closed reactor. If the reactor is an open reactor, it may be opened at the top end. If the reactor 10 is a closed reactor, it may be closed at the top end with a suitable closing device, for example a lid. If the reactor is a closed reactor, the inner volume may be under a controlled atmosphere (separate from the surroundings) or alternatively may not be under a controlled atmosphere, preferably the inner volume is not under a controlled atmosphere.

The reactor 10 comprises at least one inlet point 16, 17, preferably from one to eight inlet points, more preferably from one to five inlet points. The at least one inlet point 16 may be located at the half top portion of the reactor 10. An inlet point may be located at the open top end of the reactor, it may be an opening in the wall of the reactor or, if present, it may be an opening in the closing device. The aqueous fluid may be added into the reactor 10 at the inlet point 16.

The reactor 10 comprises at least one outlet point 18, preferably from one to three outlet points, more preferably one or two outlet points. The at least one outlet point 18 may be located at the half bottom portion of the reactor 10. An outlet point 18 may be located at the bottom end of the reactor 10. The diluted aqueous fluid may be discharged at the outlet point 18.

The apparatus 1 comprises at least one recircularization loop 11, preferably from one to three recircularization loops, more preferably one recircularization loop.

The at least one recircularization loop 11 coveys the aqueous liquid as a recirculated aqueous stream from the outlet point 18 of the reactor 10 back to the reactor 10 via the inlet point 17. The recircularization loop 11 may comprise any suitable devices, which would be fluidly connected with each other, using any suitable connecting lines.

The recircularization loop 11 comprises a pump 15. The pump 15 may be located at any suitable position of the recircularization loop 11. The pump 15 may be a lobe pump. It is advantageous to use a lobe pump, as it avoids generating a shearing stress, which may impair the obtained emulsion. In contrast, the pump 15 preferably is not a roller pump, a propeller pump or a centrifugal pump. These types of pumps may generate a shearing stress, which may impair the obtained emulsion.

The apparatus 1 comprises a heat exchanger 12. The heat exchanger 12 may be located at any suitable position of the recircularization loop 11. The heat exchanger 12 may be a plate heat exchanger. It is advantageous to use a heat exchanger 12, as it allows closely controlling the temperature of the recirculated aqueous stream into the recircularization loop 11, and avoids any unwanted cooling, which may occur during the recircularization.

The pump 15 may be located upstream the heat exchanger 12. In this embodiment, the reactor is fluidly connected to the pump, the pump is fluidly connected to the heat exchanger, and the heat exchanger is fluidly connected to the reactor. Alternatively, the pump may be located downstream the heat exchanger. In this embodiment, the reactor is fluidly connected to the heat exchanger, the heat exchanger is fluidly connected to the pump, and the pump is fluidly connected to the reactor. The pump 15 is preferably located upstream the heat exchanger 12.

The apparatus 1 may also comprise at least one additional device. For example, the apparatus may additionally comprise at least one static mixer 19, at least one homogenizer 14, at least one by-pass line, at least one heater 13, at least one stirring tool 20, at least one feedling line 21, 22, at least one inlet point 16, 17 and at least one outlet point.

The apparatus 1 may comprise at least one static mixer 19. The at least one static mixer 19 may be located at any suitable position of the recircularization loop 11. It is advantageous to use a static mixer 19, as it avoids any phase dissociation to happen during the recircularization, which may lead to an inhomogeneous recirculated aqueous stream to be sent back to the reactor 10.

The apparatus may comprise at least one homogenizer 14. The at least one homogenizer 14 may be located at any suitable position of the recircularization loop 11. The at least one homogenizer 14 may be a rotor-stator homogenizer. It is advantageous to use a homogenizer 14, particularly a rotor-stator homogenizer, as it allows an efficient injection of the concentrated aqueous feed stream into the recirculated aqueous stream.

The apparatus may comprise at least one flow metering device 24. The at least one flow metering device 24 may be located at any suitable position of the recircularization loop 11. It is advantageous to use a flow metering device 24 for measuring the flow rate of the recirculated aqueous stream, thereby monitoring that the recircularization is efficiently implemented, and that the pump 15 appropriately works.

In one example, if the recircularization loop 11 comprises a pump 15, a heat exchanger 12, a static mixer 19 and a homogenizer 14, the fluid may circulate (from upstream to downstream), via the connecting lines, from the reactor 10 through the pump 15, the heat exchanger 12, the static mixer 19, and the homogenizer 14, back to the reactor 10. Alternatively, suitable configurations are also possible.

The apparatus 1 may comprise at least one by-pass line. A by-pass line is a line allowing by-passing at least one device. The by-pass line may be located at any suitable position of the recircularization loop 11. In such case, the devices located upstream and downstream the by-passed device would be directly, fluidly connected to each other. The recircularization loop 11 may comprise a line by-passing the at least one heat exchanger 12. The recircularization loop 11 may comprise a line by-passing the at least one static mixer 19, if present. The recircularization loop 11 may comprise a line both by-passing the heat exchanger 12 and the static mixer 19. The recircularization loop 11 may comprise a line by-passing the homogenizer 14, if present.

The apparatus 1 may comprise at least one heater 13. The heater 13 may be located inside the inner volume of the reactor, or it may be located within the wall thickness of the reactor, or it may be located between the walls of the reactor (in case of a double-wall reactor), or it may be located outside the wall of the reactor. The heater 13 allows heating the fluid contained within the reactor 10.

The apparatus 1 may comprise a stirring tool 20, a drive motor 26 and optionally a shaft 25. The stirring tool 20 may be located inside the reactor 10. The stirring tool 20 is associated with the drive motor 26 and the optional shaft 25. For example, the stirring tool 20 may be propeller stirring tool.

The apparatus 1 may comprise at least one first feeding line 21, 22, preferably from one to eight first feeding lines, more preferably from one to five first feeding lines. Each first feeding line may be connected to the corresponding inlet point 16 of the reactor 10 or of the recircularization loop 11. It is advantageous to provide an apparatus 1 comprising more than one feeding line 21, 22, as it allows feeding different compounds to the aqueous fluid from different lines. Particularly, using separate feeding lines facilitates the sequential addition of different compounds, as it avoids connecting the feeding line to different sources of compounds. It also avoids the cross-contamination of the different sources of compounds. It also avoids having to clean the feeding line between the feeding of different compounds. It is also advantageous providing various inlet points in the reactor 10 and in the recircularization loop 11, as it allows feeding different compounds at different points of the apparatus 1, and therefore select the most suited location for feeding a compound, depending on its properties and its compatibility with the aqueous fluid and other compounds. It eases the cleaning of the apparatus 1, as any cleaning fluid may be fed at various point of the flow path.

The apparatus 1 comprises at least one second feeding line 27. The at least one second feeding line 27 may be directly, fluidly connected to the homogenizer 14, if present. In contrast, if present, the at least one second feeding line 27 is not directly, fluidly connected to any connection lines and/or other devices of the recircularization loop 11.

If present, the at least one second feeding 27 line comprises an additional pump 28. It is advantageous to equip the second feeding line 27 with a pump 28 being separate from the pump 15 equipping the recircularization loop 11. Indeed, this allows controlling the injection of the feed stream, in an independent manner from the recirculated aqueous stream, thereby imparting an appropriate pressure and flow rate to the feed stream. The pump 28 may be a lobe pump. It is advantageous to use a lobe pump, as it avoids generating a shearing stress, which may impair the concentrated aqueous feed stream. In contrast, the pump 28 preferably is not a roller pump, a propeller pump or a centrifugal pump, as these types of pumps may generate a shearing stress, which may impair the concentrated aqueous feed stream.

If present, the at least one second feeding line may comprise an additional flow metering device 29. It is advantageous to use a flow metering device 29 for measuring the flow rate of the feed stream, thereby monitoring that the injection of the feed stream is efficiently implemented, and that the additional pump 28 appropriately works.

The apparatus 1 may comprise at least one outlet line 23. The outlet line 23 allows discharging the personal care composition obtained with the method according to the presently claimed invention. The at least one outlet line may be directly connected to an outlet point of the reactor. The direct connection of the outlet line allows separating the fluid flow during the mixing steps (through the recircularization loop) and the fluid flow once the mixing is achieved (through the outlet line). Alternatively, the at least one outlet line 23 may be branched on the recircularization loop 11. Branching the outlet line 23 on the recircularization loop 11 is advantageous in that it allows deviating the fluid flow once the mixing is achieved.

The apparatus may comprise at least one storage tank. A storage tank may be connected to the at least one outlet line. The storage tank may be permanently or temporarily connected to the at least one outlet line. After being prepared in the reactor and the recircularization loop, the personal care composition may be stored in at least one storage tank. The aqueous fluid may be stored before being filled into a suitable packaging article, for example a bottle.

The apparatus may comprise valves 30, 31, 32 at any suitable locations.

### Advantages

The presently claimed invention offers the advantage of providing a method allowing preparing a personal care composition having a stable gel network, and into which any suitable cosmetically acceptable compounds can be added, including thermosensitive compounds.

### Examples

It is provided an apparatus 1, which is configured as follows:
The apparatus 1 comprises a reactor 10, which comprises one circumferential outer wall, delimitating an inner volume. The reactor 10 is substantially cylindrical in shape, has a substantially circular cross-section and is oriented in a vertical position. The reactor 10 is a closed reactor, which is closed at the top end by a lid. The reactor 10 comprises one inlet point 16, which is located at the half top portion of the reactor 10. A feeding line 21 opens to the corresponding inlet point 16, for feeding the fluid with suitable compounds. The reactor 10 is therefore in fluid communication with the source of an aqueous fluid, which essentially consists of water (i.e., about 100 wt.-%), and any additional suitable compounds. The reactor 10 also comprises one outlet point 18, which is located at the half bottom portion of the reactor 10. A heater 13 is located inside the inner volume of the reactor 10, for heating the fluid contained therein. A propeller stirring tool 20 is arranged in the inner volume of the reactor 10, for stirring the fluid contained therein. The stirring tool 20 is driven by a drive motor 26, through a shaft 25.

The apparatus 1 comprises a recircularization loop 11, which comprises from upstream to downstream, a lobe pump 15, a plate heat exchanger 12, a static mixer 19 and a rotor-stator homogenizer 14. The recircularization loop 11 also comprises a flow metering device 24. Two adjacent devices are fluidly connected using a suitable connecting line. The recircularization loop 11 is fluidly connected to the corresponding outlet point 18 of the reactor 10 and opens to the corresponding inlet point 17 of the reactor 10. The recircularization loop 11 allows generating a loop of the aqueous fluid - thereby forming a recirculated aqueous stream - from the reactor 10 back to the reactor 10, for improving the mixing of the aqueous fluid with various compounds, and for obtaining a formulation having a satisfactory structure and satisfactory properties.

The apparatus 1 also comprises a feeding line 27, which comprises a lobe pump 28. The feeding line 27 is directly, fluidly connected to the roto-stator homogenizer 14. The feeding line 27 also comprises a flow metering device 29. The feeding line 27 is in fluid communication with the source of the concentrated aqueous feed stream, which comprises sodium laureth-2 sulfate (SLE2S) at a concentration in the range ≥ 68 to ≤ 72 wt.%, related to the total weight of the concentrated aqueous feed stream.

It is provided a method for preparing a personal care composition, which is implemented as follows:
The method comprises the step of providing an aqueous fluid and conveying it as a recirculated aqueous stream (step of providing an aqueous fluid). The aqueous fluid essentially consists of water (i.e., about 100 wt.-%). Alternatively, the aqueous fluid comprises water and at least one additional compound, which is suitable for incorporation into a personal care composition. The aqueous fluid, which is hold in the reactor 10 and which circulates as the recirculated aqueous stream, is provided at a temperature in the range of ≥ 70 to ≤ 85 °C using the heater 13 and/or the heat exchanger 12.

The method further comprises the step of providing a concentrated aqueous feed stream and directly introducing it into the recirculated aqueous stream (step of providing a concentrated aqueous feed stream). The concentrated aqueous feed stream comprises sodium laureth-2 sulfate at a concentration in the range of ≥ 68 to ≤ 72 wt.%, related to the total weight of the concentrated aqueous feed stream. The concentrated aqueous feed stream has a flow rate in the range of ≥ 35 to ≤ 65 kg/min. The concentrated aqueous feed stream has a temperature in the range of ≥ 18 to ≤ 25 °C. The concentrated aqueous feed stream is introduced into the recirculated aqueous stream for a period in the range of ≥ 3 to ≤ 30 min. The pressure of the concentrated aqueous feed stream is at least 0.4×10⁵ Pa higher than the pressure of the recirculated aqueous stream. Sodium laureth sulfate is directly introduced into the recirculated aqueous stream using a rotor-stator homogenizer 14. Once the concentrated aqueous feed stream has been added into the recirculated aqueous stream, the ratio of the weight concentrations of sodium laureth sulfate between the concentrated aqueous stream and the diluted aqueous stream is in the range of ≥ 4.0:1.0 to ≤ 5.0:1.0.

The method further comprises the step of adding the at least one fatty alcohol and the at least one non-ionic surfactant into the aqueous fluid (step of adding fatty alcohols and non-ionic surfactants). This step allows obtaining an ungelled aqueous fluid, and ungelled recirculated aqueous stream thereof. The at least one fatty alcohol is selected from the group consisting of cetyl alcohol, stearyl alcohol, cetearyl alcohol and behenyl alcohol. The at least one non-ionic surfactant is selected from the group consisting of ceteareth-25 and steareth-20. The recirculated aqueous stream, and the personal care composition thereof, comprise the at least one fatty alcohol and the at least one non-ionic surfactant in a weight ratio in the range of ≥ 10.0:1.5 to ≤ 10.0:3.0. The at least one fatty alcohol is present in the recirculated aqueous stream, such that the personal care composition obtained thereof comprises the at least one fatty alcohol in an amount in the range of ≥ 4 to ≤ 10 wt.-%, related to the total weight of the composition. The at least one non-ionic surfactant is present in the recirculated aqueous stream, such that the personal care composition obtained thereof comprises at least one non-ionic surfactant in an amount in the range of ≥ 1.0 to ≤ 2.5 wt.-%, related to the total weight of the composition.

The method further comprises the step of pre-cooling the ungelled aqueous fluid (precooling step) using the heater 13 and/or the heat exchanger 12 at a temperature in the range of ≥ 50 to ≤ 65 °C.

The method further comprises the step of providing a recirculated aqueous stream comprising at least one fatty alcohol and at least one non-ionic surfactant (step A - step of providing a gelled recirculated aqueous stream), which are comprised, in the recirculated aqueous stream and the personal care composition obtained thereof, in part or all, in a gel network system.

The method further comprises the step of exchanging heat between the recirculated aqueous stream and a cooling media, for obtaining a cooled, recirculated aqueous stream (step B - cooling step). The recirculated aqueous stream is cooled to a temperature in the range of ≥ 25 to ≤ 45°C.

The method further comprises the step of adding at least one thermosensitive compound into the cooled, recirculated aqueous stream (step C - thermosensitive addition step).

The method further comprises the step of obtaining a personal care composition (step D - final step).

Variations and modifications of the invention and further embodiments thereof, in addition to those described herein, will become apparent to those skilled in the art from the full contents of this document. The subject matter herein contains information, exemplification and guidance that can be adapted to the practice of this invention in its various embodiments and equivalents thereof. It is intended that the appended claims cover all such variations, modifications, embodiments and equivalents.

## Claims

1. A method for preparing a personal care composition, comprising at least the steps of:
(A) providing a recirculated aqueous stream comprising at least one fatty alcohol and at least one non-ionic surfactant; wherein the fatty alcohol and the non-ionic surfactant are comprised, in the recirculated aqueous stream, in part or all, in a gel network system;
(B) exchanging heat between the recirculated aqueous stream and a cooling media, for obtaining a cooled, recirculated aqueous stream; wherein the stream is cooled to a temperature of ≤ 45°C, preferably in the range of ≥ 15 to ≤ 45°C, more preferably in the range of ≥ 25 to ≤ 45°C;
(C) adding at least one thermosensitive compound into the cooled, recirculated aqueous stream; and
(D) obtaining a personal care composition.

2. A method according to claim 1, wherein the recirculated aqueous stream is recirculated through the reactor 10.

3. A method according to any preceding claims, wherein the at least one fatty alcohol is selected from the group consisting of linear or branched C₁₄ to C₃₀ fatty alcohols; more preferably from the group consisting of linear C₁₄ to C₃₀ fatty alcohols; even more preferably from the group consisting of cetyl alcohol, stearyl alcohol, cetearyl alcohol and behenyl alcohol.

4. A method according to any preceding claims, wherein the at least one non-ionic surfactant is selected from the group consisting of polyoxyethylene C₈ to C₃₀ alkyl ethers; preferably from the group consisting of polyoxyethylene C₈ to C₃₀ alkyl ethers having at least 2 ethylene oxide units, or at least 5 ethylene oxide units; more preferably from the group consisting of polyoxyethylene C₈ to C₃₀ alkyl ethers having from 10 to 50 ethylene oxide units; even more preferably from the group consisting of polyoxyethylene C₈ to C₃₀ alkyl ethers having from 15 to 30 ethylene oxide units; still preferably from the group consisting of ceteareth-25 and steareth-20.

5. A method according to any preceding claims, wherein the recirculated aqueous stream and the personal care composition obtained thereof comprise the at least one fatty alcohol and the at least one non-ionic surfactant in a weight ratio in the range of ≥ 10.0:0.5 to ≤ 10.0:4.0, more preferably in the range of ≥ 10.0:1.0 to ≤ 10.0:3.5; even more preferably in the range of ≥ 10.0:1.5 to ≤ 10.0:3.0.

6. A method according to any preceding claims, wherein the personal care composition obtained thereof comprises the at least one fatty alcohol in an amount in the range of ≥ 2.5 to ≤ 20.0 wt.-%, preferably in the range of ≥ 3 to ≤ 15 wt.-%, more preferably in the range of ≥ 4 to ≤ 10 wt.-%, related to the total weight of the composition.

7. A method according to any preceding claims, wherein the personal care composition obtained thereof comprises the at least one non-ionic surfactant in an amount in the range of ≥ 0.2 to ≤ 5.0 wt.-%, more preferably in the range of ≥ 0.5 to ≤ 4 wt.-%, even more preferably in the range of ≥ 1.0 to ≤ 2.5 wt.-%, related to the total weight of the composition.

8. A method according to any preceding claims, wherein the recirculated aqueous stream comprising at least one fatty alcohol and at least one non-ionic surfactant is provided at a temperature in the range of ≥ 50 to ≤ 65 °C.

9. A method according to any preceding claims, further comprising, before step (A), the step of adding the at least one fatty alcohol and the at least one non-ionic surfactant into the aqueous fluid.

10. A method according to claim 10, wherein the aqueous fluid has at a temperature in the range of ≥ 70 to ≤ 85 °C.

11. A method according to any preceding claims, further comprising, after adding the at least one fatty alcohol and the at least one non-ionic surfactant into the aqueous fluid, and before providing the recirculated aqueous stream of step (A), the step of pre-cooling the recirculated aqueous stream, preferably at a temperature in the range of ≥ 50 to ≤ 65 °C.

12. A method according to any preceding claims, further comprising the step of providing a concentrated aqueous feed stream and directly introducing it into the recirculated aqueous stream, preferably wherein the concentrated aqueous feed stream comprises sodium laureth sulfate.

13. A personal care composition, which is obtained with the method according to any preceding claims.

14. A personal care composition according to claim 13, being selected from the group consisting of shampoo compositions and conditioning compositions.

15. An apparatus 1, suitable for preparing a personal care composition according to claims 13 or 14, wherein the apparatus 1 comprises:
- a reactor 10 for holding an aqueous liquid; and
- a recircularization loop 11 for conveying the aqueous fluid as a recirculated aqueous stream from the reactor 10 back to the reactor 10; wherein the recircularization loop 11 comprises a pump 15 and a heat exchanger 12.

## Amended claims

### Amended claims in accordance with Rule 137(2) EPC.

1. A method for preparing a personal care composition, comprising at least the steps of:
(A) providing an apparatus 1 comprising: a reactor 10; and a recircularization loop 11; wherein the recircularization loop 11 comprises a pump 15 and a heat exchanger 12;
(B) providing an aqueous liquid, which is held in the reactor 10;
wherein the aqueous liquid is heated;
(C) conveying the aqueous fluid as a recirculated aqueous stream from the reactor 10 back to the reactor 10 through the recircularization loop 11;
wherein the recirculated aqueous stream comprises at least one fatty alcohol and at least one non-ionic surfactant;
wherein the at least one non-ionic surfactant is different from the at least one fatty alcohol;
wherein the fatty alcohol and the non-ionic surfactant form, in the recirculated aqueous stream, in part or all, a gel network system;
wherein the at least one fatty alcohol is present in the recirculated aqueous stream, such that the personal care composition obtained thereof comprises the at least one fatty alcohol in an amount in the range of ≥ 2.5 to ≤ 20.0 wt.-% related to the total weight of the composition;
wherein the at least one non-ionic surfactant is present in the recirculated aqueous stream, such that the personal care composition obtained thereof comprises at least one non-ionic surfactant in an amount in the range of ≥ 0.2 to ≤ 5.0 wt.-% related to the total weight of the composition;
(D) exchanging heat between the recirculated aqueous stream and a cooling media, for obtaining a cooled, recirculated aqueous stream; wherein the recirculated aqueous stream is cooled to a temperature of ≤ 45°C, preferably in the range of ≥ 15 to ≤ 45°C, more preferably in the range of ≥ 25 to ≤ 45°C;
(E) adding at least one thermosensitive compound into the cooled, recirculated aqueous stream; and
(F) obtaining a personal care composition.

2. A method according to claim 1, wherein the at least one fatty alcohol is selected from the group consisting of linear or branched C₁₄ to C₃₀ fatty alcohols; more preferably from the group consisting of linear C₁₄ to C₃₀ fatty alcohols; even more preferably from the group consisting of cetyl alcohol, stearyl alcohol, cetearyl alcohol and behenyl alcohol.

3. A method according to any preceding claims, wherein the at least one non-ionic surfactant is selected from the group consisting of polyoxyethylene C₈ to C₃₀ alkyl ethers; preferably from the group consisting of polyoxyethylene C₈ to C₃₀ alkyl ethers having at least 2 ethylene oxide units, or at least 5 ethylene oxide units; more preferably from the group consisting of polyoxyethylene C₈ to C₃₀ alkyl ethers having from 10 to 50 ethylene oxide units; even more preferably from the group consisting of polyoxyethylene C₈ to C₃₀ alkyl ethers having from 15 to 30 ethylene oxide units; still preferably from the group consisting of ceteareth-25 and steareth-20.

4. A method according to any preceding claims, wherein the recirculated aqueous stream and the personal care composition obtained thereof comprise the at least one fatty alcohol and the at least one non-ionic surfactant in a weight ratio in the range of ≥ 10.0:0.5 to ≤ 10.0:4.0, more preferably in the range of ≥ 10.0:1.0 to ≤ 10.0:3.5; even more preferably in the range of ≥ 10.0:1.5 to ≤ 10.0:3.0.

5. A method according to any preceding claims, wherein the personal care composition obtained thereof comprises the at least one fatty alcohol in an amount in the range of ≥ 2.5 to ≤ 20.0 wt.-%, preferably in the range of ≥ 3 to ≤ 15 wt.-%, more preferably in the range of ≥ 4 to ≤ 10 wt.-%, related to the total weight of the composition

6. A method according to any preceding claims, wherein the personal care composition obtained thereof comprises the at least one non-ionic surfactant in an amount in the range of ≥ 0.2 to ≤ 5.0 wt.-%, more preferably in the range of ≥ 0.5 to ≤ 4 wt.-%, even more preferably in the range of ≥ 1.0 to ≤ 2.5 wt.-%, related to the total weight of the composition.

7. A method according to any preceding claims, wherein the recirculated aqueous stream comprising at least one fatty alcohol and at least one non-ionic surfactant is provided at a temperature in the range of ≥ 50 to ≤ 65 °C.

8. A method according to any preceding claims, further comprising, before step (A), the step of adding the at least one fatty alcohol and the at least one non-ionic surfactant into the aqueous fluid.

9. A method according to any preceding claims, wherein the aqueous fluid has at a temperature in the range of ≥ 70 to ≤ 85 °C.

10. A method according to any preceding claims, further comprising, after adding the at least one fatty alcohol and the at least one non-ionic surfactant into the aqueous fluid, and before providing the recirculated aqueous stream of step (A), the step of precooling the recirculated aqueous stream, preferably at a temperature in the range of ≥ 50 to ≤ 65 °C.

11. A method according to any preceding claims, further comprising the step of providing a concentrated aqueous feed stream and directly introducing it into the recirculated aqueous stream, wherein the concentrated aqueous feed stream comprises sodium laureth sulfate at a concentration in the range of ≥ 60 to ≤ 80 wt.%, preferably in the range of ≥ 65 to ≤ 75 wt.%, more preferably in the range of ≥ 68 to ≤ 72 wt.%, related to the total weight of the concentrated aqueous feed stream.
